(19) Europäisches Patentamt
European Patent Office
Office européen des brevets

(11) Veröffentlichungsnummer: **0 339 345 A2**

# (12) EUROPÄISCHE PATENTANMELDUNG

(21) Anmeldenummer: **89106265.5**

(22) Anmeldetag: **08.04.89**

(51) Int. Cl.⁴: **C07D 213/72 , A23K 1/16 , //C07D213/75**

(30) Priorität: **23.04.88 DE 3813839**

(43) Veröffentlichungstag der Anmeldung: **02.11.89 Patentblatt 89/44**

(84) Benannte Vertragsstaaten: **BE CH DE ES FR GB IT LI NL**

(71) Anmelder: **BAYER AG**

**D-5090 Leverkusen 1 Bayerwerk(DE)**

(72) Erfinder: **Lindel, Hans, Dr.**
**Carl-Duisberg-Strasse 321**
**D-5090 Leverkusen 1(DE)**
Erfinder: **Hallenbach, Werner, Dr.**
**Kleiststrasse 10**
**D-4018 Langenfeld(DE)**
Erfinder: **Berschauer, Friedrich, Prof. Dr.**
**Claudiusweg 9**
**D-5600 Wuppertal 1(DE)**
Erfinder: **Klotz, Gernot, Dr.**
**Förster-Sons-Strasse 32**
**D-5653 Leichlingen 1(DE)**
Erfinder: **Greife, Heinrich A., Dr.**
**Salmstrasse 23**
**D-4018 Langenfeld(DE)**

## (54) 4-Brom-6-chlor-5-amino-2-pyridyl-ethanolamine, Verfahren zu ihrer Herstellung und ihre Verwendung als Leistungsförderer.

(57) Die vorliegende Erfindung betrifft 4-Brom-6-chlor-5-amino-2-pyridyl-ethanolamine der Formel (I)

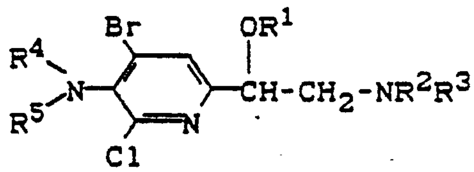

(I)

in welcher
$R^1$ für Wasserstoff, Alkyl, Acyl oder substituiertes Silyl steht,
$R^2$ für Wasserstoff oder Alkyl steht,
$R^3$ für Wasserstoff, Alkyl, Cycloalkyl, substituiertes Alkyl, gegebenenfalls substituiertes Aralkyl, Aryl oder Heterocyclyl steht,
$R^2$ und $R^3$ können gemeinsam mit dem Stickstoffatom, an das sie gebunden sind, für einen gegebe nenfalls substituierten heterocyclischen Rest stehen,
$R^4$ für Wasserstoff oder Alkyl steht,
$R^5$ für Wasserstoff, Alkyl, Halogenalkyl, Acyl steht,
sowie ihre physiologisch verträglichen Salze und ihre N-Oxide,
ihre Herstellung und ihre Verwendung als Leistungsförderer für Tiere.



Xerox Copy Centre

**4-Brom-6-chlor-5-amino-2-pyridyl-ethanolamine, Verfahren zu ihrer Herstellung und ihre Verwendung als Leistungsförderer**

Die vorliegende Erfindung betrifft 4-Brom-6-chlor-5-amino-2-pyridyl-ethanolamine, Verfahren zu ihrer Herstellung, Zwischenprodukte dafür und ihre Verwendung als Leistungsförderer für Tiere.

Die Verwendung von Futtermittelzusätzen zur Erzielung höherer Gewichtszuwächse und verbesserter Futterausnutzung wird in der Tierernährung insbesondere bei der Mast von Schweinen, Rindern und Geflügel bereits weitgehend praktiziert.

1. Es wurden neue 4-Brom-6-chlor-5-amino-2-pyridyl-ethanolamine der Formel (I) gefunden

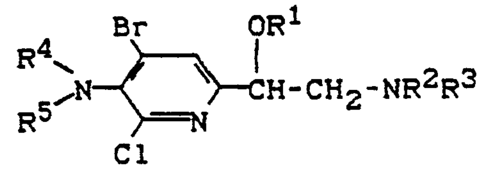
(I)

in welcher
$R^1$ für Wasserstoff, Alkyl, Acyl oder substituiertes Silyl steht,
$R^2$ für Wasserstoff oder Alkyl steht,
$R^3$ für Wasserstoff, Alkyl, Cycloalkyl, substituiertes Alkyl, gegebenenfalls substituiertes Aralkyl, Aryl oder Heterocyclyl steht,
$R^2$ und $R^3$ können gemeinsam mit dem Stickstoffatom, an das sie gebunden sind, für einen gegebenenfalls substituierten heterocyclischen Rest stehen,
$R^4$ für Wasserstoff oder Alkyl steht,
$R^5$ für Wasserstoff, Alkyl, Halogenalkyl, Acyl steht,
sowie ihre physiologisch verträglichen Salze und ihre N-Oxide.

2. Es wurde ein Verfahren zur Herstellung der 4-Brom-6-chlor-5-amino-2-pyridyl-ethanolamine der Formel (I)

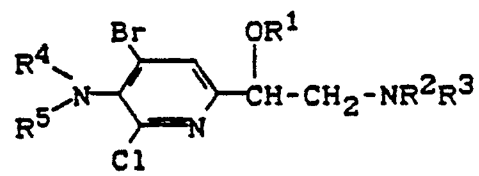
(I)

in welcher
$R^1$ für Wasserstoff, Alkyl, Acyl oder substituiertes Silyl steht,
$R^2$ für Wasserstoff oder Alkyl steht,
$R^3$ für Wasserstoff, Alkyl, Cycloalkyl, substituiertes Alkyl, gegebenenfalls substituiertes Aralkyl, Aryl oder Heterocyclyl steht,
$R^2$ und $R^3$ können gemeinsam mit dem Stickstoffatom, an das sie gebunden sind, für einen gegebenenfalls substituierten heterocyclischen Rest stehen,
$R^4$ für Wasserstoff oder Alkyl steht,
$R^5$ für Wasserstoff, Alkyl, Halogenalkyl, Acyl steht,
gefunden, das dadurch gekennzeichnet ist, daß man beta-Chlorethylverbindungen der Formel (II)

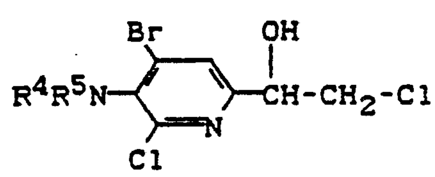
(II)

in welcher
$R^4$, $R^5$ die oben angegebene Bedeutung haben,

mit Aminen der Formel (III)
$HNR^2R^3$ (III)
in welcher
$R^2$ und $R^3$ die oben angegebene Bedeutung haben,
umsetzt und anschließend gegebenenfalls alkyliert, acyliert oder silyliert.

3. 4-Brom-5-amino-6-chlor-2-pyridyl-monochlorethanol der Formel (II)

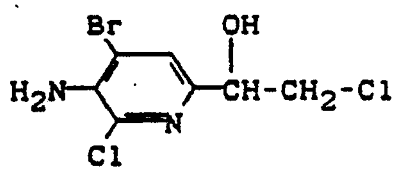

(II)

ist neu.

4. Verfahren zur Herstellung von 4-Brom-5-amino-6-chlor-2-pyridylmonochlorethanol der Formel (II) gemäß 3 (oben), dadurch gekennzeichnet, daß man 4-Brom-5-amino-6-chlor-2-chloracetyl-pyridin der Formel (IV)

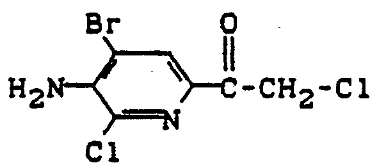

(IV)

reduziert.

5. Die Verbindung der Formel (IV)

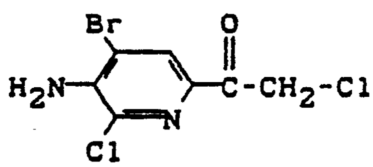

(IV)

ist neu.

6. Verfahren zur Herstellung der Verbindung der Formel (IV) gemäß 5 (oben), dadurch gekennzeichnet, daß man die Verbindung der Formel (V)

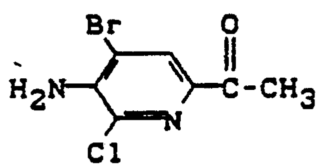

(V)

chloriert.

7. Die Verbindung der Formel (V)

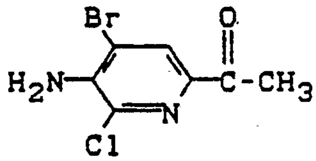

(V)

ist neu.

8. Verfahren zur Herstellung der Verbindung der Formel (V) gemäß 7 (oben), dadurch gekennzeichnet, daß man die Verbindung der Formel (VI)

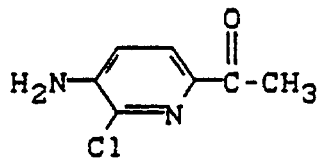 (VI)

bromiert.

9. Die Verbindung der Formel (VI)

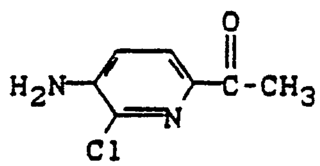 (VI)

ist neu.

10. Verfahren zur Herstellung der Verbindung der Formel (VI) gemäß 9 (oben), dadurch gekennzeichnet, daß man die Verbindung der Formel (VII)

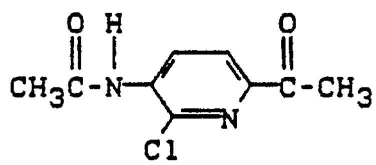 (VII)

hydrolysiert.

11. Die Verbindung der Formel (VII)

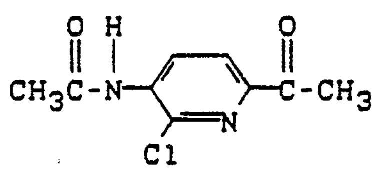 (VII)

ist neu.

12. Verfahren zur Herstellung der Verbindung der Formel (VII) gemäß 11 (oben), dadurch gekennzeichnet, daß man die Verbindung der Formel (VIII)

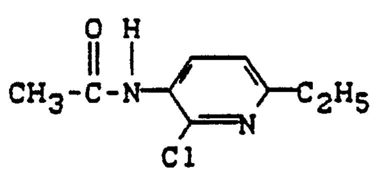 (VIII)

oxidiert.

13. Die Verbindung der Formel (VIII)

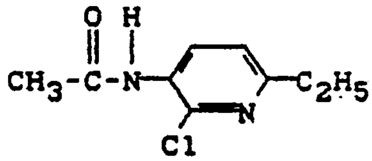

(VIII)

ist neu.

14. Verfahren zur Herstellung der Verbindung der Formel (VIII) gemäß 13 (oben), dadurch gekennzeichnet, daß man die Verbindung der Formel (IX)

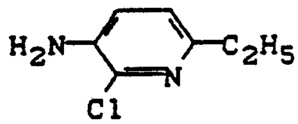

(IX)

acetyliert.

15. Die Verbindung der Formel (IX)

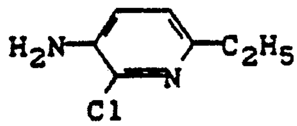

(IX)

ist neu.

16. Verfahren zur Herstellung der Verbindung der Formel (IX) gemäß 15 (oben), dadurch gekennzeichnet, daß man die Verbindung der Formel (X)

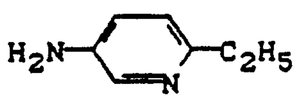

(X)

chloriert.

Die Verbindungen der Formel (I) dienen als Leistungsförderer bei Tieren. Sie besitzen insbesondere eine Wirkung zur Verschiebung des Fleisch/Fett-Verhältnisses zugunsten von Fleisch und lassen sich damit in der Tierzucht und Tierernährung einsetzen.

Die Verbindungen der Formel (I) können in Form ihrer Racemate oder enantiomeren Formen vorliegen.

Physiologisch verträgliche Salze der Verbindungen der Formel I können mit folgenden Säuren gebildet werden:

Salzsäure, Schwefelsäure, Phosphorsäure, Perchlorsäure, Brom-, Iodwasserstoffsäure, Salpetersäure, Essigsäure, Oxalsäure, Malonsäure, Bernsteinsäure, Ascorbinsäure, Äpfelsäure, Weinsäure, Maleinsäure, Fumarsäure, Methansulfonsäure, Benzoesäure, substituierte Benzoesäuren, Ameisensäure, Toluolsulfonsäure, Benzolsulfonsäure, Phthalsäure, Naphthalinsulfonsäure, Nikotinsäure, Palmitinsäure, Embonsäure.

Bevorzugt sind Verbindungen der Formel (I)

in welcher

$R^1$ für Wasserstoff, $C_{1-6}$-Alkyl oder für Carbonyl-$C_{1-6}$-alkyl, gegebenenfalls substituiertes Carbonylphenyl, Sulfonyl-$C_{1-6}$-alkyl, gegebenenfalls substituiertes Sulfonylphenyl oder für Tris-($C_1$-$C_{12}$-alkyl)-silyl, Diphenyl-$C_{1-6}$-alkyl-silyl steht,

$R^2$ für Wasserstoff oder $C_{1-6}$-Alkyl steht,

$R^3$ für Wasserstoff, gegebenenfalls substituiertes verzweigtes $C_{3-6}$-Alkyl, $C_{3-6}$-Cycloalkyl, $C_{1-6}$-Alkylphenyl steht,

$R^2$ und $R^3$ können gemeinsam mit dem Stickstoffatom, an das sie gebunden sind, für einen gegebenenfalls substituierten 5- bis 6-gliedrigen gesättigten oder ungesättigten heterocyclischen Rest stehen, der gegebenenfalls noch weitere Heteroatome aus der Reihe N, O, S enthalten kann,

$R^4$ für Wasserstoff oder $C_{1-6}$-Alkyl steht,

$R^5$ für Wasserstoff, $C_{1-6}$-Alkyl, $C_{1-4}$-Halogenalkyl, $C_{1-6}$-Alkylcarbonyl, gegebenenfalls substituiertes Benzoyl, $C_{1-6}$-Alkylsulfonyl, gegebenenfalls substituiertes Phenylsulfonyl steht.

Als Substituenten der gegebenenfalls substituierten Reste kommen bevorzugt infrage: Cyano, Halogen wie Fluor oder Chlor, Hydroxy, $C_{1-4}$-Alkyl, $C_{1-4}$-Halogenalkyl, Phenyl, $C_{1-4}$-Alkoxy, $C_{1-4}$-Halogenalkoxy, $C_{1-4}$-Alkylthio, $C_{1-4}$-Halogenalkylthio, für den Fall, daß die Substituenten an einem Phenylrest sitzen, zusätzlich bevorzugt Methylendioxy, Ethylendioxy, halogensubstituiertes Methylendioxy, halogensubstituiertes Ethylendioxy, ferner Phenyl, Phenoxy, die ihrerseits einen oder mehrere der obengenannten Substituenten tragen können.

Besonders bevorzugt sind Verbindungen der Formel (I)

in welcher

$R^1$ für Wasserstoff, $C_{1-6}$-Alkyl, insbesondere Methyl oder Ethyl steht,

$R^2$ für Wasserstoff oder $C_{1-4}$-Alkyl, insbesondere Methyl oder Ethyl steht,

$R^3$ für Wasserstoff, gegebenenfalls durch 1 bis 5 Halogenatome substituiertes verzweigtes $C_{3-6}$-Alkyl, insbesondere i-Propyl, t-Butyl, Pentyl, Hexyl, wobei insbesondere sekundäre und tertiäre Alkylreste genannt seien, sowie $C_{1-6}$-Alkylphenyl, das im Phenylring gegebenenfalls durch Halogen, insbesondere Fluor oder Chlor, $C_{1-4}$-Alkyl, Hydroxy, $C_{1-4}$-Alkoxy, gegebenenfalls halogensubstituiertes Methylendioxy oder Ethylendioxy substituiert sein kann, steht

$R^4$ für Wasserstoff steht,

$R^5$ für Wasserstoff steht.

Im einzelnen seien folgende Verbindungen der Formel (I) genannt:

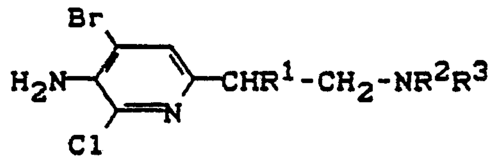

| $R^1$ | $R^2$ | $R^3$ |
|---|---|---|
| OH | H | $i\text{-}C_3H_7$ |
| $OCH_3$ | H | $i\text{-}C_3H_7$ |
| OH | H | $t\text{-}C_4H_9$ |
| OH | H | $t\text{-}C_4H_8F$ |
| OH | H | 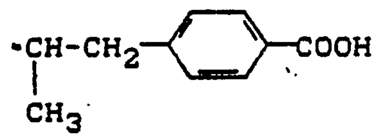 |
| OH | H | 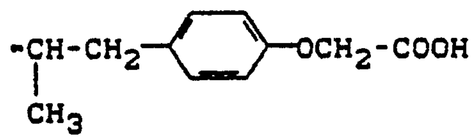 |
| OH | H | 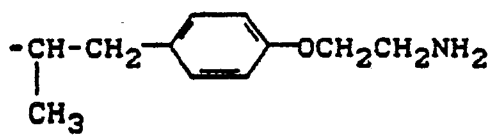 |
| OH | H | 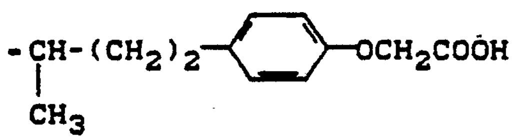 |
| OH | H | 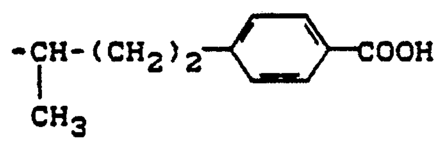 |

Bevorzugt seien die Salze mit Salzsäure, Schwefelsäure, Phosphorsäure, Oxalsäure, Maleinsäure, Fumarsäure, Malonsäure genannt.

Setzt man zur Herstellung der 4-Brom-6-chlor-5-amino-2-pyridyl-ethanolamine als beta-Halogenethylverbindung der Formel (II) 4-Brom-5-amino-6-chlor-2-(1-hydroxy-2-chlorethyl)-pyridin und als Amin der Formel (III) t-Butylamin ein, läßt sich das Verfahren zur Herstellung der Verbindung der Formel (I) durch folgendes Formelschema wiedergeben:

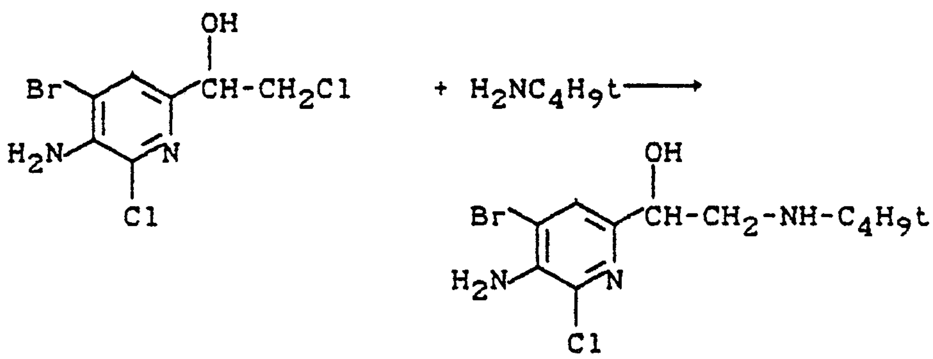

Beta-Halogenethylverbindungen der Formel (II) sind neu. Ihre Herstellung wird weiter unten beschrieben.

Im einzelnen seien folgende Verbindungen der Formel (II) genannt:

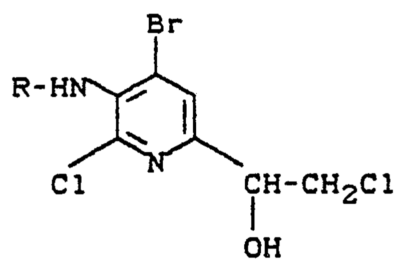

R = 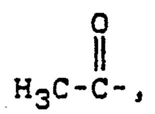, $H_3CSO_2-$, H

Als Amine der Formel (III) seien bevorzugt genannt:
Ammoniak, Methylamin, Dimethylamin, Ethylamin, Diethylamin, Methylethylamin, n-Propylamin, i-Propylamin, n-Butylamin, i-Butylamin, tert.-Butylamin, Cyclopentylamin, Cyclohexylamin, Benzylamin, Anilin, Piperidin, Pyrrolidin, Morpholin, 2-Aminopyridin, 3-(4-Carbomethoxyphenyl)-2-propylamin, 3-(4-Methoxycarbonylmethoxyphenyl)-2-propylamin, 3-(4-Carboxyphenyl)-2-propylamin, 3-(4-Carboxymethoxyphenyl)-2-propylamin, 3-(4-Hydroxymethylphenyl)-2-propylamin, 3-(4-(2-Hydroxyethyl)-phenyl)-2-propylamin.

Das Verfahren wird durchgeführt, indem man die beta-Halogenethylverbindung der Formel (II) mit überschüssigem Amin der Formel (III) gegebenenfalls in Gegenwart eines Verdünnungsmittels umsetzt.

Amin der Formel (III) wird in 2-10-molarem, bevorzugt 2-3-molarem Überschuß eingesetzt.

Die Reaktion wird bei Temperaturen von +20 bis +250 °C, bevorzugt zwischen 50 und 150 °C, durchgeführt.

Es wird bei Normaldruck oder, vor allem bei flüchtigen Aminen, unter erhöhtem Druck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Cyclohexan, Benzol, Toluol, Methylenchlorid, Chloroform, ferner Ether wie Diethylether, Tetrahydrofuran und Dioxan, weiterhin Nitrile wie Acetonitril und Benzonitril, ferner Amide wie Dimethylformamid, ferner Alkohole wie Methanol, Ethanol, n- und i-Propanol.

Bevorzugt eingesetzt werden Alkohole.

Die Aufarbeitung erfolgt, indem überschüssiges Amin sowie Lösungsmittel abdestilliert wird. Die Reinigung der Verbindungen erfolgt durch Kristallisation.

Verfahren 4 zur Herstellung der Verbindungen der Formel (II) läßt sich durch folgendes Formelschema wiedergeben:

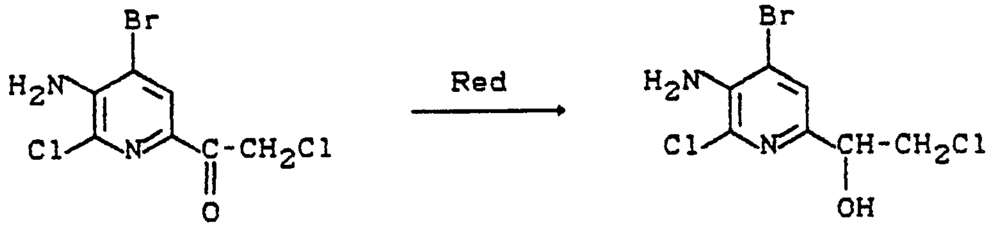


Das Verfahren wird durchgeführt, indem man die Verbindung der Formel (IV) in einem Verdünnungsmittel mit dem Reduktionsmittel umsetzt.

Als Reduktionsmittel dienen komplexe Hydride wie Natriumborhydrid, Lithiumborhydrid, außerdem Diboran.

Die Reaktion wird bei Temperaturen von -20 °C bis +100 °C durchgeführt.

Es wird bevorzugt bei Normaldruck gearbeitet.

Als Verdünnungsmittel dienen alle inerten organischen Lösungsmittel. Hierzu zählen insbesondere gegebenenfalls halogenierte aliphatische und aromatische Kohlenwasserstoffe wie Pentan, Hexan, Cyclohexan, Benzol, Toluol, Methylenchlorid, Chloroform, Chlorbenzol; Ether wie Diethylether, Tetrahydrofuran; Nitrile wie Acetonitril, Benzonitril; Alkohole wie Methanol, Ethanol, n- und i-Propanol. Bevorzugt werden Alkohole eingesetzt.

Verfahren 6 zur Herstellung der Verbindung der Formel (IV) läßt sich durch folgendes Formelschema wiedergeben:

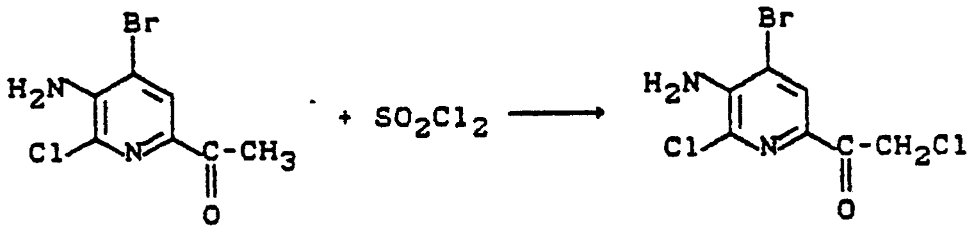

Das Verfahren wird durchgeführt, indem man pro Mol der Verbindung der Formel (V) in einem Verdünnungsmittel 1-3 Mol, bevorzugt 1-1,5 Mol, Halogenierungsmittel zugibt.

Als Halogenierungsmittel dienen Sulfurylchlorid, Chlor, N-Chlorverbindungen wie z.B. N-Chlorsuccinimid. Bevorzugt ist Sulfurylchlorid.

Die Reaktion wird zwischen -20 °C bis +150 °C durchgeführt, bevorzugt zwischen 0 °C und 70 °C.

Es wird bevorzugt bei Normaldruck gearbeitet.

Als Verdünnungsmittel seien genannt: aliphatische, gegebenenfalls halogenierte Kohlenwasserstoffe wie Pentan, Hexan, Heptan, Cyclohexan, Methylenchlorid, Ethylenchlorid, Chloroform, Tetrachlorkohlenstoff, Alkohole wie Methanol, Ethanol, Ester wie Essigsäureethylester und Mischungen aus diesen Verdünnungsmitteln.

Verfahren 8 zur Herstellung der Verbindung der Formel (V) läßt sich durch folgendes Formelschema wiedergeben:

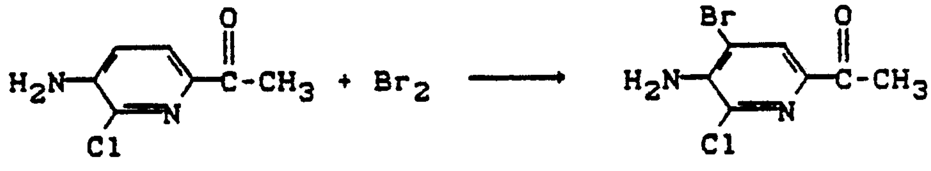

Das Verfahren wird durchgeführt, indem man die Verbindung der Formel (VI) in Gegenwart eines Verdünnungsmittels mit Brom oder einem Bromierungsmittel umsetzt.

Es wird bei Temperaturen zwischen -20 °C und +150 °C, bevorzugt zwischen 0 °C und 70 °C, gearbeitet.

Auf 1 Mol Verbindung der Formel (VI) wird in etwa die äquivalente Menge Brom oder Bromierungsmittel

eingesetzt.

Als Verdünnungsmittel seien genannt aliphatische, gegebenenfalls halogenierte Kohlenwasserstoffe wie z.B. Pentan, Hexan, Heptan, Cyclohexan, Methylenchlorid, Ethylenchlorid, Chloroform, Tetrachlorkohlenstoff, Alkohole wie Methanol, Ethanol, Ester wie Essigsäureethylester, Eisessig.

Die Aufarbeitung erfolgt in üblicher Weise z.B. durch Abdestillieren des Verdünnungsmittels.

Verfahren 10 zur Herstellung der Verbindung der Formel (VI) läßt sich durch folgendes Formelschema wiedergeben:

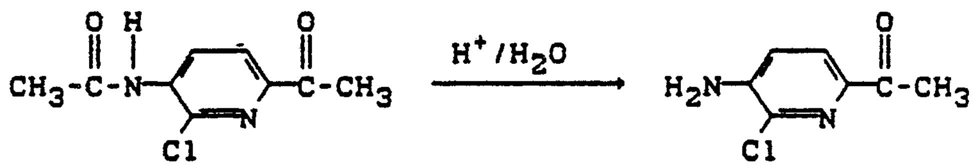


Das Verfahren wird durchgeführt, indem man die Verbindung der Formel (VII) gegebenenfalls in Gegenwart eines Verdünnungsmittels mit Säuren behandelt.

Als Säuren dienen HCl, $H_2SO_4$, HBr, $H_3PO_4$, Methansulfonsäure, Benzolsulfonsäure oder stark saure Ionenaustauscher.

Als Verdünnungsmittel dienen Wasser sowie mit Wasser mischbare Lösungsmittel wie z.B. Alkohole wie Methanol, Ethanol, i-Propanol, Ketone wie Aceton. Bevorzugt sind Alkohole.

Es wird bei Temperaturen zwischen 0 und 150 °C, bevorzugt zwischen 50 und 120 °C, gearbeitet.

Es wird bevorzugt bei Normaldruck gearbeitet.

Die Aufarbeitung erfolgt nach Neutralisation durch Abfiltrieren oder Extrahieren der Verbindung der Formel (VI).

Verfahren 12 zur Herstellung der Verbindung der Formel (VII) läßt sich durch folgendes Formelschema wiedergeben:

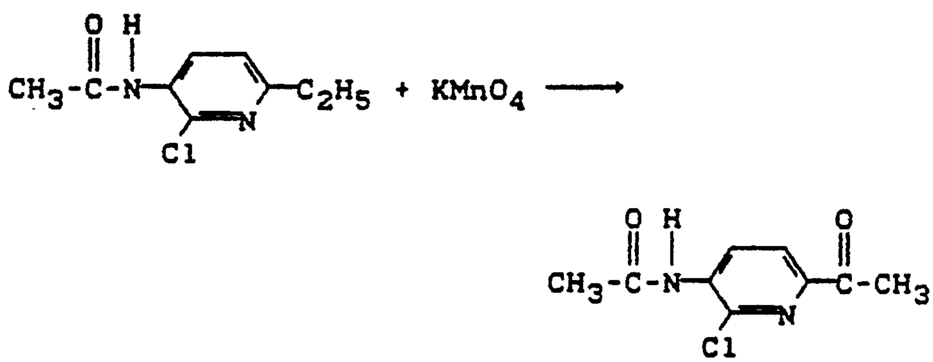


Das Verfahren wird durchgeführt, indem man die Verbindung der Formel (VIII) in wasserhaltigen Verdünnungsmitteln in Gegenwart von Puffersubstanzen mit Kaliumpermanganat oxidiert.

Als Verdünnungsmittel dient Wasser im Gemisch mit Alkoholen wie z.B. t-Butanol, Ketonen wie z.B. Aceton, Aminen wie z.B. Pyridin, Nitrilen wie z.B. Acetonitril, Ethern wie z.B. Diglykoldimethylether.

Die Reaktion wird bei pH-Werten zwischen 4 und 10, bevorzugt zwischen pH 5 und 8, durchgeführt. Diese pH-Werte werden entweder durch laufende Zudosierung einer Säure wie z.B. Essigsäure eingestellt, oder sie werden in Gegenwart von Puffersubstanzen wie z.B. $NaH_2PO_4$, $Mg(NO_3)_2$ bzw. $MgO/HNO_3$, $MgSO_4$, $CaSO_4$, $NaHCO_3$ eingehalten.

Es werden 1,1-6 Mol $KMnO_4$ und 1,1-6 Mol Puffersubstanz, bevorzugt jeweils 1,1-3 Mol pro Mol Verbindung der Formel (VIII) eingesetzt.

Es wird bei Temperaturen zwischen 0 und 70 °C, bevorzugt zwischen 10 und 30 °C, gearbeitet.

Es wird bei Normaldruck gearbeitet.

Die Aufarbeitung erfolgt, indem man vom Braunstein abfiltriert und das Lösungsmittel des Filtrats abdestilliert.

Verfahren 14 zur Herstellung der Verbindung der Formel (VIII) läßt sich durch folgendes Formelschema darstellen:

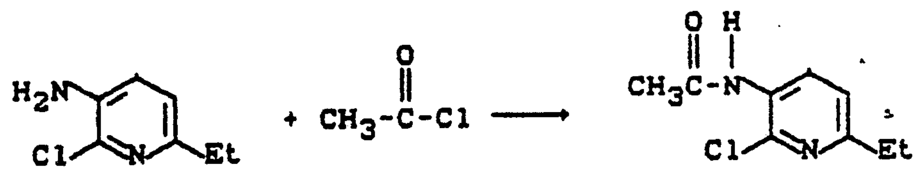

Als Acylierungsmittel dienen: Acetanhydrid, Acetylchlorid, Keten.

Das erfindungsgemäße Verfahren kann mit oder ohne Verdünnungsmittel durchgeführt werden. Als Verdünnungsmittel kommen praktisch alle inerten organischen Lösungsmittel in Frage. Hierzu gehören insbesondere aliphatische und aromatische, gegebenenfalls halogenierte Kohlenwasserstoffe wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone wie Aceton, Methylethyl-, Methylisopropyl- und Methylisobutylketon, außerdem Ester wie Essigsäure-methylester und -ethylester, ferner Nitrile wie z.B. Acetonitril und Propionitril, Benzonitril, Glutarsäuredinitril, darüber hinaus Amide wie z.B. Dimethylformamid, Dimethylacetamid und N-Methylpyrrolidon sowie Tetramethylensulfon und Hexamethylphosphorsäuretriamid.

Zur Beschleunigung des erfindungsgemäßen Verfahrens können Katalysatoren und Hilfsbasen zugesetzt werden. Als solche sind geeignet: z.B. Na-Acetat, tertiäre Amine wie Pyridin, 4-Dimethylaminopyridin, Triethylamin, Triethylendiamin, Trimethylen-tetrahydropyrimidin; ferner Zinn-II- und Zinn-IV-Verbindungen wie Zinn-II-octoat oder Zinn-IV-chlorid. Die als Reaktionsbeschleuniger genannten tertiären Amine, z.B. Pyridin, können auch als Lösungsmittel verwendet werden.

Die Reaktion wird bei Temperaturen von 0 bis 250 °C, bevorzugt von 50 bis 150 °C, durchgeführt.

Die Reaktion wird bevorzugt bei Normaldruck durchgeführt.

Es werden 1-5 Mol, bevorzugt 1-2 Mol, Acylierungsmittel pro Mol Verbindung der Formel (IX) eingesetzt. Es werden 1-5 Mol, bevorzugt 1-2 Mol, Hilfsbase pro Mol der Verbindung (IX) eingesetzt. Falls verwendet, werden 0,01-1 Mol Katalysatoren pro Mol Verbindung der Formel (IX) eingesetzt.

Das Reaktionsgemisch wird mit Wasser verdünnt, extrahiert oder abfiltriert, gegebenenfalls wird das Lösungsmittel abdestilliert.

Verfahren 16 zur Herstellung der Verbindung der Formel (X) läßt sich durch folgendes Formelschema darstellen:

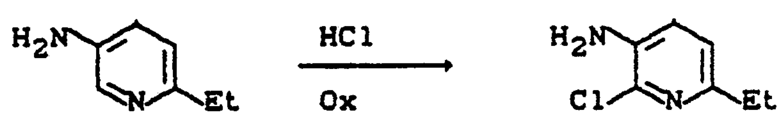


Die Umsetzung erfolgt mit Salzsäure in Gegenwart eines Oxidationsmittels.

Als Oxidationsmittel dienen bevorzugt $H_2O_2$, dessen Salze und Derivate wie z.B. Natriumperoxid, Persäuren wie aliphatische und aromatische Persäuren. Als solche seien genannt: Perpropionsäure, Perbenzoesäure, die gegebenenfalls substituiert sein können. Weitere Oxidationsmittel sind Salze oder Ester der Hypochlorigen Säure wie z.B. Natriumhypochlorit, sowie Kaliumpermanganat, Mangandioxid, Peroxomono- und Peroxodisulfate.

Es werden 1-4 Äquivalente, besonders bevorzugt 1-1,5 Äquivalente Oxidationsmittel pro Mol Verbindung (X) eingesetzt.

Die Reaktion wird bevorzugt in wäßriger Lösung durchgeführt. Sie kann auch in organischen Lösungsmitteln, die gegenüber den Reaktionsbedingungen inert sind, durchgeführt werden.

Das Verfahren wird bei Temperaturen zwischen 0 und 150 °C, bevorzugt zwischen 10 und 100 °C, besonders bevorzugt zwischen 20-80 °C durchgeführt.

Es wird bei Normaldruck gearbeitet.

Die Chlorierung kann jedoch auch mit Sulfurylchlorid durchgeführt werden.

5-Amino-3-ethylpyridin ist bekannt. Es kann analog zu dem von G.H. Cooper et al., J. Chem. Soc. C (1971), 3257-60 beschriebenen Verfahren hergestellt werden, indem man die entsprechende Nitroverbindung reduziert. Die Reduktion erfolgt dabei in wasserhaltigen Lösungsmittelgemischen mit Wasserstoff und Palladium-Aktivkohle-Katalysator.

Die Wirkstoffe eignen sich als Mittel zur Leistungsförderung bei Zucht- und Nutztieren. Sie dienen

dabei zur Förderung und Beschleunigung des Wachstums, der Milch- und Wollproduktion, sowie zur Verbesserung der Futterverwertung, der Fleischqualität und zur Verschiebung des Fleisch-Fett-Verhältnisses zugunsten von Fleisch.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale.

Die Wirkstoffe werden unabhängig vom Geschlecht der Tiere während allen Wachstums- und Leistungsphasen der Tiere eingesetzt. Bevorzugt werden die Wirkstoffe während der intensiven Wachstums- und Leistungsphase eingesetzt. Die intensive Wachstums- und Leistungsphase dauert je nach Tierart von einem Monat bis zu 10 Jahren. Besonders wertvoll erweisen sich die Wirkstoffe bei Aufzucht und Haltung von Jung- und Masttieren.

Die Anwendung der Wirkstoffe erfolgt direkt oder in Form von für Tiere geeigneten Zubereitungen enteral oder parentereal. Die enterale Anwendung der Wirkstoffe geschieht z.B. oral in Form von Pulvern, Tabletten, Kapseln, Pasten, Tränken, Granulaten, Boli, über oral applizierbare Lösungen, Emulsionen oder Suspensionen sowie über das Futter oder über das Trinkwasser. Die parenterale Anwendung geschieht z.B. in Form der Injektion (intramuskulär, subcutan, intravenös oder durch Implantate).

Besonders hervorgehoben seien Zubereitungen zur Verabreichung über das Futter oder das Trinkwasser. Dabei können die Wirkstoffe dem Futter direkt oder in Form von Prämixen oder Futterkonzentraten zugesetzt werden.

Zum Futter zählen Einzelfuttermittel pflanzlicher Herkunft wie Heu, Rüben, Getreide und Getreidenebenprodukte, Melasse, Silage, Einzelfuttermittel tierischer Herkunft wie Fleisch, Fette, Milchprodukte, Knochenmehl, Fischprodukte, die Einzelfuttermittel wie Vitamine, Proteine, Zucker, Stärke, Mehle, Aminosäuren z.B. DL-Methionin, Salze wie Kalk und Kochsalz. Zum Futter zählen auch Ergänzungs-, Fertig-und Mischfuttermittel. Diese enthalten Einzelfuttermittel in einer Zusammensetzung, die eine ausgewogene Ernährung hinsichtlich der Energie- und Proteinversorgung sowie der Versorgung mit Vitaminen, Mineralsalzen und Spurenelementen sicherstellt.

Prämixe und Futterkonzentrate sind Mischungen des Wirkstoffs mit Trägerstoffen und gegebenenfalls weiteren Hilfsstoffen. Zu den Trägerstoffen zählen alle Einzelfuttermittel oder Gemische derselben.

Die Wirkstoffe können in den Zubereitungen allein oder in Mischung mit anderen leistungsfördernden Wirkstoffen, mineralischen Futtermitteln, Spurenelement-Verbindungen, Vitaminen, stickstoffhaltigen nicht-Protein-Verbindungen, Farbstoffen, Antioxidantien, Aromastoffen, Emulgatoren, Fließhilfsstoffen, Konservierungs- und Preßhilfsstoffen vorliegen.

Andere leistungsfördernde Wirkstoffe sind z.B. Antibiotika wie Tylosin und Virginiamycin.

Mineralische Futtermittel sind z.B. Dicalciumphosphat, Magnesiumoxid, Natriumchlorid.

Spurenelement-Verbindungen sind z.B. Eisenfumarat, Natriumjodid, Kobaltchlorid, Kupfersulfat, Zinkoxid, Selenverbindungen.

Vitamine sind z.B. Vitamin A, Vitamin $D_3$, Vitamin E.

Stickstoffhaltige nicht-Protein-Verbindungen sind z.B. Biuret, Harnstoff.

Farbstoffe sind z.B. Carotinoide wie Canthaxanthin, Zeaxanthin, Capsanthin oder Farbstoffe, die zur Färbung von Lebensmitteln zugelassen sind.

Antioxidantien sind z.B. Ethoxyquin, Butylhydroxy-Toluol, Ascorbinsäure.

Aromastoffe sind z.B. Vanillin.

Emulgatoren sind z.B. Ester der Milchsäure, Lecithin. Fließhilfsstoffe sind z.B. Natriumstearat, Calciumstearat, Kieselsäuren, Bentonite, Ligninsulfonate.

Konservierungsstoffe sind z.B. Propionsäure, Calciumpropionat, Sorbinsäure, Ascorbinsäure.

Preßhilfsstoffe sind z.B. Ligninsulfonate, Celluloseether.

Die Konzentration der Wirkstoffe im Futter beträgt normalerweise etwa 0,001-500 ppm, bevorzugt 0,1-50 ppm.

Die Konzentration der Wirkstoffe in den Prämixen oder Futterkonzentraten beträgt ca. 0,5 bis 50 Gewichtsprozent, bevorzugt 1 bis 20 Gewichtsprozent.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 50 mg/kg insbesondere 0,01 bis 5 mg/kg Körpergewicht pro Tag. Die passende Menge des Wirkstoffs sowie die passende Dauer der Verabreichung hängen insbesondere von der Art, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab.

Die Wirkstoffe können einmalig verabreicht werden. Die Wirkstoffe können aber auch während der ganzen oder während eines Teils der Wachstums- und Leistungsphase temporär oder kontinuierlich verabreicht werden. Bei kontinuierlicher Verabreichung kann die Anwendung ein-oder mehrmals täglich in regelmäßigen oder unregelmäßigen Abständen erfolgen.

Beispiel für die Zusammensetzung eines Kükenaufzuchtfutters, das erfindungsgemäßen Wirkstoff enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung der unten angegebenen Zusammensetzung und 2,5 g Wirkstoff Prämix der unten angegebenen Zusammensetzung ergeben nach sorgfältigem Mischen 1 kg Futter.

In einem kg Vitamin-Mineral-Mischung sind enthalten: 600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg Mn $SO_4$ x $H_2O$, 140 mg Zn $SO_4$ x 7 $H_2O$, 100 mg Fe $SO_4$ x 7 $H_2O$ und 20 mg Cu $SO_4$ x 5 $H_2O$ in Getreidemehl als Trägerstoff.

1 kg Wirkstoff-Prämix enthalten 100 g Wirkstoff, 900 g Weizenmehl.

Beispiel für die Zusammensetzung eines Schweineaufzuchtfutters, das erfindungsgemäßen Wirkstoff enthält:

630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Bierhefe, 50 g Vitamin-Mineral-Mischung (Zusammensetzung wie beim Kükenfutter) 30 g Leinkuchenmehl, 30 g Maiskleberfutter, 10 g Sojaöl, 10 g Zuckerrohrmelasse und 2 g Wirkstoff-Prämix ergeben nach sorgfältigem Mischen 1 kg Futter.

1 kg Wirkstoff-Prämix enthält 200 g Wirkstoff, 20 g Pflanzenöl, 780 g Calciumcarbonatpulver.

Beispiel für die Zusammensetzung eines Futters für Rinder, das den erfindungsgemäßen Wirkstoff enthält: 69,95 % Futtergetreideschrot, 10 % gemahlene Maiskolben, 8 % Sojabohnenmehl, 5 % Luzernemehl, 5 % Melasse, 0,6 % Harnstoff, 0,5 % Calciumphosphat, 0,5 % Calciumcarbonat, 0,3 % Kochsalz, 0,15 % Vitamin-Mineral-Mischung und 0,2 % Wirkstoff-Prämix der beim Schweineaufzuchtfutter angegebenen Zusammensetzung. Die Vitamin-Mineral-Mischung enthält pro kg 70.000 i.E. Vitamin A, 70.000 i:E. Vitamin $D_3$, 100 mg Vitamin E, 50 mg Mn $SO_4$ x $H_2O$, 30 mg Zn $SO_4$ x $7H_2O$ in Getreidemehl als Trägerstoff.

Der Wirkstoff-Prämix wird der Vitamin-Mineral-Mischung in der erforderlichen Menge beigemischt und diese anschließend sorgfältig mit den übrigen Bestandteilen vermischt.

## Beispiel

Ratten-Fütterungsversuch

Weibliche Laborratten 90-110 g schwer vom Typ SPF Wistar (Züchtung Hagemann) werden ad lib mit Standard Rattenfutter, das mit der gewünschten Menge Wirkstoff versetzt ist, gefüttert. Jeder Versuchsansatz wird mit Futter der identischen Charge durchgeführt, so daß Unterschiede in der Zusammensetzung des Futters die Vergleichbarkeit der Ergebnisse nicht beeinträchtigen können.

Die Ratten erhalten Wasser ad lib.

Jeweils 12 Ratten bilden eine Versuchsgruppe und werden mit Futter, das mit der gewünschten Menge Wirkstoff versetzt ist gefüttert. Eine Kontrollgruppe erhält Futter ohne Wirkstoff. Die Versuchsgruppen werden so zusammengestellt, daß das durchschnittliche Körpergewicht sowie die Streuung in den Körpergewichten der Ratten in jeder Versuchsgruppe gleich ist, so daß eine Vergleichbarkeit der Versuchsgruppen untereinander gewährleistet ist.

Die Tiere werden vor Versuchsbeginn 2 Tage an die neuen Haltungsbedingungen adaptiert, während dieser Zeit wird Futter ohne Wirkstoffzusatz gegeben. Danach erhalten die Tiere 13 Tage lang Futter, das Wirkstoff enthält. Es wird die relative Gewichtszunahme im Verhältnis zur unbehandelten Kontrolle bestimmt.

Es werden die aus der Tabelle ersichtlichen Ergebnisse erhalten:

Tabelle

| Ratten-Fütterungsversuch | | |
|---|---|---|
| Wirkstoff Bsp.-Nr. | Wirkstoffkonzentration ppm | relative Gewichtszunahme |
| 5 | 25 | 123 |
| 6 | 25 | 119 |
| 10 | 25 | 120 |
| 3 | 25 | 113 |
| 4 | 25 | 128 |
| 1 | 25 | 155 |

Herstellungsbeispiele

Beispiel 1

2-N-tert.-Butylamino-1-(3-amino-4-brom-2-chlor-6-pyridyl)-ethanol

2 g (7 mmol) 2-Chlor-1-(3-amino-4-brom-2-chlor-6-pyridyl)-ethanol und 1,28 g (17,5 mmol) tert.-Butylamin werden in 10 ml $CHCl_3$ gelöst und in einem Autoklaven 12 Stunden auf 100 °C erhitzt. Zur Aufarbeitung wird zwischen verdünnter NaOH und $CH_2Cl_2$ verteilt, die organische Phase abgetrennt, getrocknet und eingedampft. Der Rückstand wird zwischen 5 %igem $NaH_2PO_4$ und Ether verteilt, die wäßrige Phase abgetrennt, mit NaOH alkalisch gestellt und dreimal mit $CH_2Cl_2$ extrahiert. Nach Trocknung mit $Na_2SO_4$ wird im Vakuum eingedampft. Der Rückstand wird in Ether gelöst und Petrolether bis zur beginnenden Trübung zugegeben. Nach Beendigung der Kristallisation wird abgesaugt und mit Petrolether gewaschen.
Ausbeute: 660 mg (29 % d.Th.)
Schmelzpunkt: 122 °C

Analog werden hergestellt:

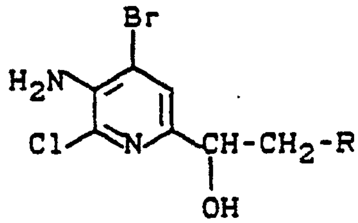

| Bsp. Nr. | R | Schmp.: (° C) |
|---|---|---|
| 2 | 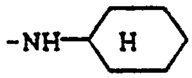 | 88 |
| 3 | 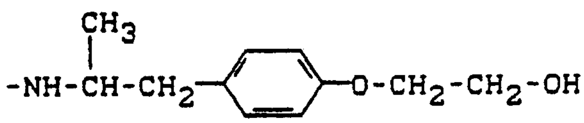 | Öl IR: 3350, 2950, 1610, 1500, 1450, 1360, 1240, 1100, 1070, 1040, 720cm$^{-1}$ |
| 4 | $-NH-CH(CH_3)_2$ | Öl IR: 3300, 2950, 1660, 1610, 1450, 1360, 1100, 1040, 870, 750 cm$^{-1}$ |
| 5 | 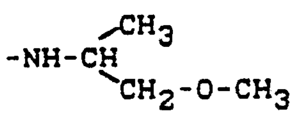 | 138 |
| 6 | 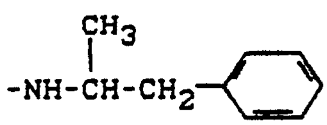 | Öl IR: 3350, 2950, 1600, 1560, 1530, 1450, 1360, 1030, 720 cm$^{-1}$ |
| 7 | $-NH-(CH_2)_3CH_3$ | 112 |

| Bsp. Nr. | R | Schmp.: (°C) |
|---|---|---|
| 8 | 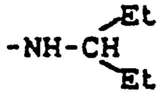 | Öl IR: 3350, 2950, 1600, 1560, 1530, 1450, 1360, 1100, 860, 750 $cm^{-1}$ |
| 9 | 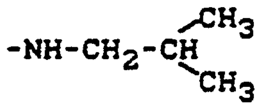 | 93 |
| 10 | 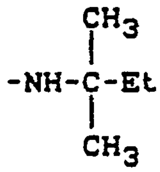 | 89 |
| 11 | 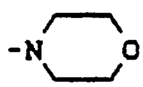 | 118 |
| 12 | 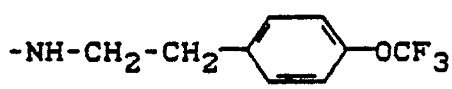 | 65 |
| 13 | 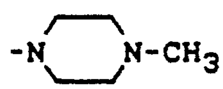 | 124 |

Beispiel für Verfahren 4 zur Herstellung der Verbindung der Formel (II).

<u>2-Chlor-1-(3-amino-4-brom-2-chlor-6-pyridyl)-ethanol</u>

1 g (3,52 mmol) 3-Amino-4-brom-2-chlor-6-chloracetylpyridin werden in 15 ml Methanol suspendiert, auf 0 °C gekühlt und 133 mg (3,52 mmol) $NaBH_4$ zugegeben. Nach 10 Minuten wird auf Wasser gegossen, überschüssiges $NaBH_4$ durch Ansäuern mit HCl zerstört und mit $NaHCO_3$ wieder neutralisiert. Nach Extraktion mit $CH_2Cl_2$ und Abdampfen des Lösungsmittels wird 1 g (99 % der Theorie) als Rückstand erhalten.
Schmelzpunkt: 30 °C

Beispiel für Verfahren 6 zur Herstellung der Verbindung der Formel (IV).

<u>3-Amino-4-brom-2-chlor-6-chloracetyl-pyridin</u>

0,8 g (3,2 mmol) 6-Acetyl-3-amino-4-brom-2-chlor-pyridin werden in 5 ml $CHCl_3$ gelöst und 462 mg (3,42 mmol) $SO_2Cl_2$, gelöst in 5 ml $CHCl_3$, zugegeben. Es wird 30 Minuten bei Raumtemperatur gerührt, dann auf $NaHCO_3$-Lösung gegossen und mit $CH_2Cl_2$ extrahiert.

Nach Abdampfen des Lösungsmittels im Vakuum verbleibt ein brauner Feststoff.
Ausbeute: 800 mg (88 % der Theorie)
Schmelzpunkt: 150° C.

Beispiel für Verfahren 8 zur Herstellung der Verbindung der Formel (V).

6-Acetyl-3-amino-4-brom-2-chlorpyridin

15 g (88 mmol) 6-Acetyl-3-amino-2-chlorpyridin werden in 88 ml Eisessig suspendiert und 4,5 ml (88 mmol) Brom zugetropft. Es wird noch 30 Minuten bei Raumtemperatur nachgerührt, dann abgesaugt und der Filterkuchen zwischen $NaHCO_3$-Lösung und $CHCl_3$ verteilt. Die organische Phase wird abgetrennt, mit $Na_2SO_4$ getrocknet und eingedampft.
Ausbeute: 13 g (60 % der Theorie)
Schmelzpunkt: 184° C

Beispiel für Verfahren 10 zur Herstellung der Verbindung der Formel (VI).

6-Acetyl-3-amino-2-chlorpyridin

5 g (24 mmol) 3-Acetamido-6-acetyl-2-chlorpyridin werden in einer Mischung aus 25 ml Konzentrierter wäßriger HCl und 25 ml Methanol eine Stunde unter Rückfluß gekocht. Nach Abkühlung wird vorsichtig auf 600 ml gesättigte $NaHCO_3$-Lösung gegeben und dreimal mit je 200 ml Dichlormethan extrahiert. Die gesammelten organischen Phasen werden mit $Na_2SO_4$ getrocknet und eingedampft. Der Rückstand wird aus Toluol/Petrolether umkristallisiert.
Ausbeute: 3,4 g (84,5 % der Theorie)
Schmp.: 137° C gelbe Kristalle.

Beispiel für Verfahren 12 zur Herstellung der Verbindung der Formel (VII).

6-Acetyl-3-acetamido-2-chlorpyridin

4,937 g (0,124 mol) MgO werden in 112 ml Wasser suspendiert und mit ca. 16,7 ml (0,242 mol) 65 %iger $HNO_3$ auf pH 6 gestellt. Anschließend werden 10 g (49,4 mmol) 3-Acetamido-2-chlor-6-ethylpyridin, gelöst in 112 ml Aceton, zugegeben, 19,5 g (0,124 mol) $KMnO_4$ eingetragen und bis zur vollständigen Umsetzung bei 20° C gerührt (48 Stunden). Danach wird abgesaugt, der Filterkuchen sorgfältig mit heißem Aceton gewaschen. Das Filtrat wird mit 500 ml Wasser verdünnt, dreimal mit je 200 ml $CHCl_3$ extrahiert, die vereinigten Extrakte it Wasser gewaschen, mit $Na_2SO_4$ getrocknet und eingedampft. Zur Reinigung wird aus Toluol/Petrolether umkristallisert.
Ausbeute: 8,5 g (81 % der Theorie)
Schmp.: 183° C farblose Kristalle.

Beispiel für Verfahren 14 zur Herstellung der Verbindung der Formel (VIII).

3-Acetamido-2-chlor-6-ethylpyridin

27 g (173 mmol) 3-Amino-2-chlor-6-ethylpyridin werden in 220 ml trockenem $CHCl_3$ gelöst, 24,7 ml (177 mmol) $Et_3N$ und 17,7 ml (187 mmol) Acetanhydrid zugefügt. Der Ansatz bleibt 12 Stunden bei

Raumtemperatur stehen und wird danach noch zwei Stunden unter Rückfluß erhitzt.

Zur Aufarbeitung wird auf 400 ml Wasser gegeben, die organische Phase abgetrennt und zweimal mit je 300 ml $NaHCO_3$-Lösung gewaschen. Nach Trocknung mit $Na_2SO_4$ wird eingedampft.
Ausbeute: 28 g (82 % der Theorie)
Schmp.: 104° C

Beispiel für Verfahren 16 zur Herstellung der Verbindung der Formel (IX).

3-Amino-2-chlor-6-ethylpyridin

2 g (16,4 mmol) 5-Amino-2-ethylpyridin (Herstellung G.H. Cooper und R.L. Rickard, J. Chem. Soc. C, 3257-60 (1971)) werden in 17,7 ml konzentrierter HCl gelöst und dann vorsichtig mit 2,2 ml (21,4 mol) 30 %iger $H_2O_2$-Lösung versetzt. Es tritt eine exotherme Reaktion auf. Die Temperatur wird durch Kühlung mit einem Eisbad bei etwa 50° C gehalten und nach Abklingen der Reaktion noch 15 Minuten bei dieser Temperatur nachgerührt. Zur Aufarbeitung wird auf 200 ml Wasser gegeben, mit 40 %iger Natronlauge auf pH 10 gestellt und zweimal mit je 100 ml Dichlormethan extrahiert. Nach Trocknung mit $Na_2SO_4$ wird eingedampft, der Rückstand mit Petrolether verrührt und abgesaugt.
Ausbeute: 1,6 g (62,3 % der Theorie)
Schmp.: 104° C

Beispiel zur Herstellung von 5-Amino-2-ethylpyridin:

40 g (0,263 mol) 2-Ethyl-5-nitropyridin werden in einer Mischung von 400 ml Dioxan und 50 ml Wasser gelöst, 2 g 10 % Pd/Aktivkohle zugegeben und bei Raumtemperatur und unter Normaldruck bis zur vollständigen $H_2$-Aufnahme hydriert. Es wird vom Katalysator abfiltriert und eingedampft.
Ausbeute: 32,1 g quantitativ
gelbes Öl, wird an der Luft schnell braun

**Ansprüche**

1. 4-Brom-6-chlor-5-amino-2-pyridyl-ethanolamine der Formel (I)

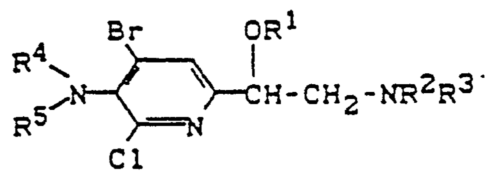

(I)

in welcher
$R^1$ für Wasserstoff, Alkyl, Acyl oder substituiertes Silyl steht,
$R^2$ für Wasserstoff oder Alkyl steht,
$R^3$ für Wasserstoff, Alkyl, Cycloalkyl, substituiertes Alkyl, gegebenenfalls substituiertes Aralkyl, Aryl oder Heterocyclyl steht,
$R^2$ und $R^3$ können gemeinsam mit dem Stickstoffatom, an das sie gebunden sind, für einen gegebenenfalls substituierten heterocyclischen Rest stehen,
$R^4$ für Wasserstoff oder Alkyl steht,
$R^5$ für Wasserstoff, Alkyl, Halogenalkyl, Acyl steht,
sowie ihre physiologisch verträglichen Salze und ihre N-Oxide.

2. Verfahren zur Herstellung der 4-Brom-6-chlor-5-amino-2-pyridyl-ethanolamine der Formel (I)

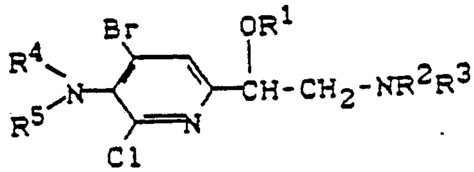

(I)

in welcher
$R^1$ für Wasserstoff, Alkyl, Acyl oder substituiertes Silyl steht,
$R^2$ für Wasserstoff oder Alkyl steht,
$R^3$ für Wasserstoff, Alkyl, Cycloalkyl, substituiertes Alkyl, gegebenenfalls substituiertes Aralkyl, Aryl oder Heterocyclyl steht,
$R^2$ und $R^3$ können gemeinsam mit dem Stickstoffatom, an das sie gebunden sind, für einen gegebenenfalls substituierten heterocyclischen Rest stehen,
$R^4$ für Wasserstoff cder Alkyl steht,
$R^5$ für Wasserstoff, Alkyl, Halogenalkyl, Acyl steht,
dadurch gekennzeichnet, daß man beta-Chlorethylverbindungen der Formel (II)

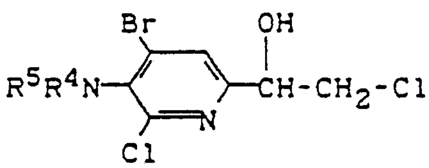

(II)

in welcher
$R^4$, $R^5$ die oben angegebene Bedeutung haben,
mit Aminen der Formel (III)
$HNR^2R^3$ (III)
in welcher
$R^2$ und $R^3$ die oben angegebene Bedeutung haben,
umsetzt und anschließend gegebenenfalls alkyliert, acyliert oder silyliert.

3. 4-Brom-5-amino-6-chlor-2-pyridyl-Verbindungen der Formel

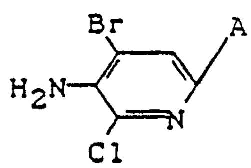

n welcher
A für die Reste
$-\overset{OH}{\overset{|}{C}}H \ -CH_2Cl$, $-\overset{O}{\overset{||}{C}}-CH_2-Cl$, $-\overset{O}{\overset{||}{C}}-CH_3$ steht.

4. Verbindungen der Formel

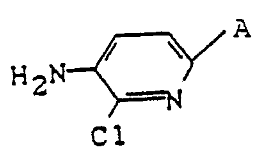

in welcher
A für die Reste
$-\overset{OH}{\overset{|}{C}}H \ -CH_2Cl$, $-\overset{O}{\overset{||}{C}}-CH_2-Cl$, $-\overset{O}{\overset{||}{C}}-CH_3$ steht.

5. Verbindungen der Formel

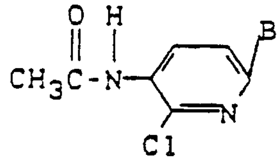

in welcher
B für die Reste
$-\overset{O}{\overset{\|}{C}}-CH_3$, $-C_2H_5$ steht.

6. Mittel zur Leistungsförderung von Tieren, gekennzeichnet durch einen Gehalt an 4-Brom-6-chlor-5-amino-2-pyridyl-ethanolamin der Formel (I) gemäß Anspruch 1.

7. Verwendung von 4-Brom-6-chlor-5-amino-2-pyridylethanolamin der Formel (I) gemäß Anspruch 1 zur Leistungsförderung von Tieren.

8. Verwendung von 4-Brom-6-chlor-5-amino-2-pyridylethanolaminen der Formel (I) gemäß Anspruch 1 zur Herstellung von Mitteln zur Leistungsförderung bei Tieren.